# EUROPEAN PATENT SPECIFICATION

(11) **EP 0 574 221 B1**
(45) Date of publication and mention of the grant of the patent: **22.11.2000**
(21) Application number: 93304427.3
(22) Date of filing: 08.06.1993
(51) Int. Cl.: A61K 31/455, A61K 47/34

(54) **Pharmaceutical stable formulations of nicorandil**
Pharmazeutische stabile Nicorandil-Formulierungen
Formulations pharmaceutiques stables à base de nicorandil

(30) Priority: 08.06.1992 IT MI921413
(43) Date of publication of application: 15.12.1993
(73) Proprietor: THERAPICON SRL, I-20146 Milan (IT)
(72) Inventor: Veronesi, Paolo Alberto, I-20146 Milano (IT); Veronesi, Anna Maria, I-06083 Bastia Umbra PG (IT)
(74) Representative: Baverstock, Michael George Douglas

(56) References cited:
- WO-A-86/06960
- FR-A- 2 624 012
- US-A- 4 837 029
- CHEMICAL ABSTRACTS, vol. 106, no. 24 Columbus, Ohio, US; abstract no. 201753d, KAMIYAMA, FUMIO, ET AL 'transdermal tape containing n-(2-hydroxyethyl)nicotinamide nitrate (ester) and siloxane adhesive' & JP-A-62 036 317

## Description

The present invention relates to pharmaceutically stable compositions comprising nicorandil and methods of producing the same. In particular the invention provides a composition for pharmaceutical use comprising a suspension or dispersion of nicorandil in polydimethyl siloxanes.

Nicorandil, the nitrate ester of N-(2-hydroxyethyl)-nicotinamide, is described in the published literature as a derivative with very good coronary vasodilative and coronary vasoconstriction suppressing actions. It has been described as being useful in the treatment of various types of angina pectoris whilst causing minimum effects on the dynamics of cardiovascular circulation and on cardiac functions. It is further described as having an inhibitory action on the coronary channels of calcium ions.

Use of nicorandil has been proposed in various forms, for example in Japanese patent application no. JP A 62036317 it is proposed to incorporate nicorandil in a transdermal tape.

In the solid crystalline state nicorandil is stable in conditions of extreme dryness, but when exposed to moisture even if only for short periods at low humidity levels and at room temperature nicorandil degrades. Three factors influence the hydrolysis of nicorandil, the increased percentage of moisture to which the product is exposed, the temperature and the storage period.

Progressive degradation of nicorandil is caused by the hydrolysis of the nitrate ester contained in the molecule. This hydrolysis results in liberation of nitric acid and of N-(2-hydroxyethyl) nicotinamide, this latter substance not being pharmacologically active at the dosages of interest, as is evidenced by the substantial decrease of the content of the active ingredient and therefore of the pharmacological activity. The degradation of nicorandil in the presence of water is so rapid that in aqueous solution at 5% the product loses at 60°C and at pH7 in only 12 hours almost 20% of its titer, whereas the powder at the dry state and at the same conditions of temperature does not exhibit during the same period appreciable quantitative variations.

Further, it has been shown that if crystalline nicorandil is compressed into a tablet form not only does it degrade through contact with moisture, although with more reduced intensity, but the degradation reaches substantially increased levels during subsequent storage periods. Friction between the crystals of the nicorandil resulting from compression during the manufacture of tablets, breaking of the crystals and the like has been described as a possible reason for the instability. The resultant instability may be caused by the crystals of nicorandil being broken during the manufacture. This argument has been described in European patent application 0 230 932.

The adoption of alternative production methods of conventional tablets of nicorandil, for example using variable quantities of saturated higher fatty acids, or of their inorganic salts, or saturated higher alcohols (having waxy or solid consistancy at room temperature), leads to slight improvements, but the stability of nicorandil, when formulated in tablets, remains relatively precarious and unsatisfactory.

The use of moisture-proof packing materials for packing tablets has been proposed as a means of preventing moisture effecting the nicorandil. However this results in increased expense, and is insufficient to prevent moisture being absorbed by the nicorandil. Further, the material does not stop degradation of the nicorandil once the packaging is opened.

In consideration of the clinico-therapeutic importance of providing a pharmaceutical product containing nicorandil in which the nicorandil is relatively stable, in other words of maintaining adequate dosages of nicorandil when formulated in pharmaceutical forms, during the periods of normal storage, we set about developing formulations of nicorandil, which would be stable for an adequate period of time, and which would be compatible with the excipients used for the formulation of pharmaceuticals and finally which could be stored without considerable degradation in conventional packing materials, for example small glass or plastic bottles with cap-screw, blisters or strips made from thermosealed coupled foils of aluminium and copolymers.

In one attempt to obtain stable preparations of nicorandil, we tried suspending nicorandil in variable quantities of triglycerides of aliphatic acid, saturated or unsaturated, of from, C-4 to C-12, linear or ramified, monohydroxylated or not, or in variable quantities of polyglycolised glycerides of aliphatic saturated or unsaturated fatty acids, having from C-8 to C-22, linear or ramified, monohydroxylated or not, or in mixtures of variable quantities of the above glycerides and polyglycolised glycerides, the above vehicles being a liquid or semi-solid state at ordinary temperatures. Hard and soft gelatin capsules, preferably with a sealing band, were filled with the above dispersions and the resulting pharmaceutical preparation showed an appreciable increase in the stability of nicorandil. Moreover, this preparation method avoids the compression of the nicorandil crystals which, as observed initially, is a possible cause of instability of nicorandil. The concentrations of nicorandil in those compositions varied from 1% to 50% by weight. The triglycerids may be individual substances or mixtures in variable proportions of conventional esters well known to those skilled in the art, characterized by the following general formula: 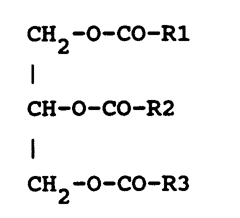 wherein R1, R2 and R3 are aliphatic fatty acids, which may vary from C-4 to C-12, for the degree of unsaturation (having most commonly from 1 to 6 double bonds), isomerism (of position, of stereospecificity or of binding). The polyglycolised glycerides are mixtures of esters, of different proportions, of conventional products, well known to those skilled in the art being esters having the following H.L.B. (Hydrophilic-Lipophilic-Balance) values:

| | H.L.B. Values |
|---|---|
| Triglycerides | from 1 to 2 |
| Diglycerides | from 2 to 3 |
| Monoglycerides | from 3 to 4 |
| Diesters of polyethylene glycol | from 6 to 15 |
| Monoesters of polyethylene glycol | from 10 to 17 |

where the proportions of the 5 ingredients are characterized from the proportion of the reaction mixture. The figures of amphophily are expressed as H.L.B. values (from 1 to 17). The main aliphatic acids most commonly esterified with the medium triglycerides (from C-8 to C-12) and with the saturated polyglycolised glycerides, are preferably, among the saturated fatty acids, lauric acid, myristic acid, palmitic acid, stearic acid and arachidic acid, among the unsaturated fatty acids, lauroleic acid, myristoleic acid, palmitoleic acid, oleic acid, linoleic acid and linolenic acid, among the ramified, isostearic acid and among the monohydroxylated, ricinoleic acid.

However, samples of the above described formulations of nicorandil stored at temperatures around 40°C developed, after an interval of some weeks, a considerable decrease in the assay of the active ingredient nicorandil. Our studies established that nicorandil interacts with the triglycerides of the fatty acids and/or with the polyglycolised glycerides of aliphatic fatty acids, by virtue of a transesterification process which leads to the hydrolysis of nicorandil with the formation of different esters of N-(2-hydroxyethyl) nicotinamide (primary aminoalcohol) with one or more fatty acids resulting from the triglyceride and/or from the polyglycolised glyceride which substances are not active pharmacologically and the simultaneous substitution of the fatty acid with nitric acid, resulting from nicorandil. This phenomenon can be analysed using high pressure liquid chromatography, which shows that as soon as the transesterification of nicorandil takes place, multiple peaks corresponding to the various resulting products, having different retention times and much higher than nicorandil itself. Other authors (Iida Yoshimitsu, European patent application 0 230 932) on the contrary adopted formulations of nicorandil tablets, in which they used as excipients considerable quantities of high aliphatic saturated acids, or of their inorganic salts, or high aliphatic saturated alcohols, solid at the ordinary temperatures, optionally added with a certain quantity of a special organic acid. The dissolution profile of those preparations containing only nicorandil with high aliphatic saturated acids or high saturated alcohols, mixed also with other conventional support excipients, is not particularly satisfactory. With the aim of improving the dissolution profile of the tablet, the same authors included into the compositions a disaggregating agent or preferably an organic acid. Despite the efforts to control the moisture content below the normal (3-5%) those tablets of nicorandil showed a certain instability, evidenced by a decrease of the assay of nicorandil during normal storage periods.

Furthermore, insufficient dissolution of nicorandil can be a disadvantage for this type of active ingredient, for which sometimes a rapid absorption and a prompt pharmacological and vasodilating effect is required. There was a need to make available preparations of nicorandil with a better stability and with a rapid bioavailability not only by oral route, but also by sublingual route which preparations were not provided by previous compositions. It was further recognised by the present inventors that the ideal pharmaceutical formulation to be used for nicorandil should also work as a protective barrier, by preventing external humidity contacting the nicorandil and the excipients which are used, so as to protect nicorandil from the external humidity. It should also be inert and chemically compatible, in order to avoid interactions which may inactivate the nicorandil, as it did for example in the above mentioned transesterification with the triglycerides of the fatty acids and/or the polyglycosated glycerides of aliphatic acids.

According to the present invention there is provided a pharmaceutical composition suitable for oral or sub-lingual administration comprising a suspension or dispersion of nicorandil in one or more polydimethyl siloxanes.

The pharmaceutical suspensions/dispersions of the present invention contain as essential active ingredient nicorandil. The suspensions/dispersions also contain polydimethyl siloxanes and as optional ingredients colloidal silica, lecithin, sweetening agent, flavouring agent and/or essence. The compositions of nicorandil of the present invention, show a surprising stability even during relatively large storage periods.

Preferably nicorandil is present in the polydimethyl siloxane suspension or dispersion in an amount of from 0.5% to 50% by weight and preferably in an amount of 5% to 20% by weight of the composition. Preferably the pharmaceutical composition is moisture free.

The polydimethyl siloxanes are chemically inert and do not interact with N-(2-hydroxyethyl)-nicotinamide nitrate ester which are hydrorepellent and provide a valuable protection barrier against exogenous humidity, both being factors which contribute to a rapid degradation of nicorandil. Moreover the polydimethyl siloxanes are resistant to heat, to the action of atmospheric agents for example oxygen, ozone, water and light, possess an appreciable absence of toxicity and a good tolerability. Preferably, before being mixed with the nicorandil any moisture within the polydimethyl siloxane is reduced to a minium for example by heating to drive off the moisture. The polydimethyl siloxanes used in the composition may comprise a mixture of polydimethyl siloxanes.

Examples of Polydimethyl siloxanes for pharmaceutical use according to the present invention (more commonly defined in the European Pharmacopoeia, volume II, page 138 as "dimethiconum" or "dimethicone" and in the French Pharmacopoeia 9.th edition as "low and medium density silicone oils") are preferably linear polymers and preferably have the following structural formula: 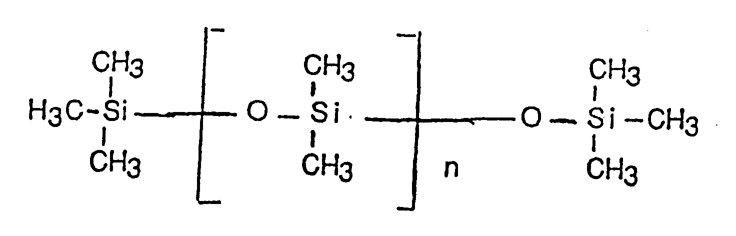 Preferably the degree of polymerization varies from 20 to 400. More preferably the degree of polymerisation is from 30 to 100. The polydimethyl siloxanes may be obtained according to known methods including through hydrolysis and polycondensation of dichloromethylsiloxane and of chlorotrimethylsilane. There are different types of polymethyl siloxanes which are differentiated by the indications of the nominal viscosity, which is represented by an indication following the name of the substance. Examples of polydimethyl siloxanes are dimethicone and simethicone which are commonly available in different grades of viscosity. Dimethicone is colourless, odourless and thus possesses ideal organoleptic characters for use in pharmaceutical compositions.

Preferred suspensions of nicorandil in polydimethyl siloxanes, giving a satisfactory and uniform distribution of the nicorandil, are preferably obtained using polydimethyl siloxanes having a viscosity of from 20 mm²/S to 1000 mm²/S preferably from 28 mm²/S to 110 mm²/S and more preferably from 40mm ²/S to 70 mm²/S and most preferably from 45 to 60 mm^{2/}S at 25°C.
Polydimethyl siloxanes with higher viscosity may cause production problems during repartition of the dispersions or suspensions.

According to a further embodiment of the present invention suspensions of nicorandil in polydimethylsiloxanes may be inserted into soft or hard gelatin capsules, said gelatin capsules constituting an additional protection barrier for the nicorandil polydimethyl siloxane suspension or dispersion especially against external moisture. The gelatin capsules may include a sealing bond.

It is appreciated that the gelatin capsules contain some moisture but preferably this is kept to a minimum. Known soft or hard gelatin capsules may be used. The capsules comprise gelatin and may further comprise for example one or more of glycerol, modified sorbitol mixtures, for example sorbitol and sorbitan mixtures (one such mixture on the market is andrisorb (trade mark) which is sorbitol/sorbitan mixture in the ratio of 85/70), titanium dioxide, preserving and colouring agents. Preferably the soft or hard gelatin capsules are of a size which enables desired doses of nicorandil to be administered. For example different size capsules may be provided which enable desired dose units of nicorandil of 5 mg, 10 mg and 20 mg to be administered.

In order to obtain an improved homogenous suspension of nicorandil in polydimethyl siloxane and improved uniformity of dosage, small quantities of colloidal silica for example aerosil (trade mark) which is a colloidal silicon dioxide, may be added so as to increase the suspendibility of the nicorandil in the polydimethyl siloxanes. Further, the colloidal silica may additionally have the advantage of preventing at least some moisture which may be present or which may enter into the suspension during storage, interacting with nicorandil, which moisture would cause possible hydrolytic degradation of the nicorandil. The percentage by weight of colloidal silica in the above composition preferably is in the range of from 0.5% to 20% and more preferably from 1% to 10%.

Further, lecithin may be included in the suspension. Preferably the lecithin acts as a suspending agent. Preferably the percentage by weight of the lecithin is from 0.5% to 20% and more preferably from 1% to 10% by weight.

Sweetening agents may be optionally added to the composition provided that they are chemically compatible with the other ingredients of the composition, in order to make the nicorandil suspension more palatable and acceptable for, for example,sublingual administration. Examples of sweetening agents include sugar, cyclamates, saccharins and aspartame. Natural or synthetic flavouring agents or essences may also optionally be added.

The pharmaceutical compositions of the present invention may be taken orally or sub-lingually. In the form of the invention wherein the nicorandil suspension/dispersion is contained in a soft or hard gelatin capsule, in order to obtain quick intake of the nicorandil said capsule may be placed in the mouth and broken for example by chewing of the teeth.

Examples of pharmaceutical compositions according to the invention are as follows:

| Composition | Ex.1 5% | Ex.2 10% | Ex.3 20% |
|---|---|---|---|
| nicorandil | 5.000 mg | 10.000 mg | 20.000 mg |
| dimethicone | 91.833 mg | 87.833 mg | 78.917 mg |
| colloidal silica | 3.167 mg | 2.167 mg | 1.083 mg |
| Total Weight | 100.000 mg | 100.000 mg | 100.000 mg |

Examples of preferred soft gelatin shells are as follows:

| | | | |
|---|---|---|---|
| gelatin | 50.400 mg | 50.400 mg | 50.400 mg |
| glycerol | 24.304 mg | 24.304 mg | 24.304 mg |
| titanium dioxide E171 | 0.799 mg | 0.799 mg | 0.300 mg |
| iron oxide red E172 | 0.020 mg | 0.200 mg | 0.799 mg |
| Weight shell | 75.523 mg | 75.703 mg | 75.803 mg |

Further details of each of the above example embodiments are given below:

### EXAMPLE 1

Preparation of 1,800 soft gelatin capsules, size 2 oval, containing 5 mg of nicorandil.

Composition of the filling suspension, corresponding to the individual content of a soft gelatin capsule dosed at 5 mg of nicorandil (5% by weight of the composition):

| | |
|---|---|
| - nicorandil | 5.000 mg |
| - dimethicone (viscosity 50 mm²/s (50 cSt) ± 5%) | 91.833 mg |
| - silica (aerosil) | 3.167 mg |
| Total weight of the composition | 100.000 mg |

Composition of the external shell:

| | |
|---|---|
| - gelatin | 50.400 mg |
| - glycerol | 24.304 mg |
| - titanium dioxide E171 | 0.799 mg |
| - iron oxide red E172 | 0.020 mg |
| Total weight of the shell | 75.523 mg |

Nicorandil (9.0 g), after sieving on a 30 mesh sieve, and silica (5.7 g) was accurately suspended in dimethicone V 50 mm²/s (50 cSt) (165.3 g) and the mixture was mixed to homegeneity. The weight of the obtained mixture was about 180.0 grams. The suspension was loaded, under constant stirring, into the feeding tank of a dosing loop for liquid suspensions of a machine for the production of soft gelatin capsules. The soft gelatin number 2, oval shape capsules were filled with the suspension following well known procedures. The soft gelatin capsules obtained (No. 1,705 / yield 94.7 %) were collected in stainless steel trays and stabilized (dehydrated) by conventional methods. The soft gelatin capsules were then preserved in amber glass bottles, with screw cap, at a temperature of 40°C for 180 days and analysed after 90 days and at the end of the storage period. Reference tablets (about 1,000 tablets), were prepared using conventional methods and the following conventional formulation:

| | |
|---|---|
| - nicorandil | 5.000 mg |
| - mannitol | 71.700 mg |
| - starch | 20.000 mg |
| - methylcellulose | 0.300 mg |
| - magnesium stearate | 3.000 mg |
| Total weight of the tablet | 100.000 mg |

Mannitol (71.7 g), starch (20.0 g), methylcellulose (0.3 g) and nicorandil (5.0 g) were accurately mixed in a polyethylene bag. Finally magnesium stearate (3.0 g) was added and after a short mixing of about 3 minutes, the mixture was compressed directly with a suitable punch in order to obtain tablets having a mean unitary weight of about 100.0 mg. The obtained reference tablets (No. 953 / yield 95.3 %) were packed in separate amber glass bottles, with screw cap, preserved at 40°C for 180 days and analysed after 90 days and at the end of the storage period.

The assay of nicorandil in the soft gelatin capsules according to the invention and in the reference tablets was determined by a suitable high pressure liquid chromatographic method, in order to determine the content of nicorandil during and at the end of the period of storage so as to compare the stability of the two formulations. The quantity of nicorandil expressed as % of the initial assay (considered as 100 %) was determined after production, but before the storage period. The results are set out in the following Table 1. The results establish that the soft gelatin capsules of the invention showed an appreciable stability during the storage period, and that the stability was much higher than the normal reference tablets, preserved at the same conditions.

**TABLE 1**

| Stability study of soft gelatin capsules and of reference tablets of Example 1, dosed at 5 mg of nicorandil, expressed as assay % of the initial figure (means figure obtained from the determination of 10 units of each pharmaceutical form). | | | |
|---|---|---|---|
| Compositions | Initial (% theoretic) | Intervals | |
| | | 90 days | 180 days |
| Soft gelatin capsules | 99.76 | 99.15 | 98.91 |
| Reference tablets | 99.08 | 85.92 | 67.24 |

### EXAMPLE 2

Preparation of 2,500 soft gelatin capsules, size 2 oval, containing 10 mg of nicorandil.

Composition of the filling suspension, corresponding to the individual content of a soft gelatin capsule dosed at 10 mg of nicorandil.

| | |
|---|---|
| - nicorandil | 10.000 mg |
| - dimethicone (viscosity 50 mm²/s (50 cSt) ± 5%) | 87.833 mg |
| - silica (aerosil) | 2.167 mg |
| Total weight of the composition | 100.000 mg |

Composition of the external shell

| | |
|---|---|
| - gelatin | 50.400 mg |
| - glycerol | 24.304 mg |
| - titanium dioxide E171 | 0.799 mg |
| - iron oxide red E172 | 0.200 mg |
| Total weight of the shell | 75.703 mg |

Nicorandil (25.0 g) after sieving on a mesh sieve, and silica (5.4 g) were accurately suspended in dimethicone V 50 mm²/s (50 cSt) (219.6 g) and the mixture was stirred to homogeneity. The weight of the obtained mixture was about 250.0 grams. The suspension was loaded, under constant stirring, into the feeding tank of a dosing loop for liquid suspensions of a machine for the production of soft gelatin capsules. The soft gelatin capsules No. 2, shape oval, were filled with the suspension following well known procedures. The soft gelatin capsules obtained (No. 2,384 / yield 95.36 %) were collected in stainless steel trays and stabilized (dehydrated) using conventional methods. The soft gelatin capsules were then preserved in amber glass bottles, with screw cap, at a temperature of 40°C for 180 days and analysed at 90 days and at the end of the storage period.

Reference tablets (about 1,000 tablets), were prepared using conventional methods and the following conventional formulation.

| | |
|---|---|
| - nicorandil | 10.000 mg |
| - mannitol | 68.000 mg |
| - starch | 10.000 mg |
| - calcium carboxymethylcellulose | 6.000 mg |
| - hydroxypropylcellulose | 3.000 mg |
| - calcium stearate | 3.000 mg |
| Total weight of the tablet | 100.00 mg |

Mannitol (68.0 g), starch (10.0 g), calcium carboxymethylcellulose (6.0 g), hydroxypropylcellulose (3.0 g) and nicorandil (10.0 g) were accurately mixed in a polyethylene bag. Finally calcium stearate (3.0 g) was added and after a short time (about 3 minutes) mixing, the mixture was compressed directly with a suitable punch in order to obtain tablets having a mean unitary weight of about 100.0 mg. The obtained reference tablets (No. 947 / yield 94.7 %) were packed in separate amber glass bottles, with screw cap, preserved at 40° C for 180 days and analysed at 90 days and at the end of the storage period.

The assay of nicorandil in the samples of soft gelatin capsules according to the invention and in the reference tablets was determined by a suitable high pressure liquid chromatographic method, in order to determine the content of active ingredient during and at the end of the storage period so as to compare the stability of the two formulations.

The quantity of nicorandil expressed as a % of the initial assay (considered as 100 %) was determined after the production but, before the storage period. The results are set out in the following Table 2. The results establish that the soft gelatin capsules of the invention showed an appreciable stability during the storage period, and that the stability was much higher than the normal reference tablets, preserved at the same conditions.

**TABLE 2**

| Stability study of soft gelatin capsules and of reference tablets of Example 2, dosed at 10 mg of nicorandil, expressed as assay % of the initial figure (mean figure obtained from the determination of 10 units of each pharmaceutical form). | | | |
|---|---|---|---|
| Compositions | Initial (% theoretic) | Intervals | |
| | | 90 days | 180 days |
| Soft gelatin capsules | 99.53 | 98.82 | 98.64 |
| Reference tablets | 99.72 | 85.16 | 69.10 |

### EXAMPLE 3

Preparation of 3,000 soft gelatin capsules, size 2 oval, containing 20 mg of nicorandil.

Composition of the filling suspension, corresponding to the individual content of a soft gelatin capsule dosed at 20 mg of nicorandil.

| | |
|---|---|
| - nicorandil | 20.000 mg |
| - dimethicone (viscosity 100 mm²/s (100 cSt) ± 5%) | 80.000 mg |
| Total weight of the composition | 100.000 mg |

Composition of the external shell

| | |
|---|---|
| - gelatin | 50.400 mg |
| - glycerol | 24.304 mg |
| - titanium dioxide E171 | 0.300 mg |
| - iron oxide red E172 | 0.799 mg |
| Total weight of the shell | 75.803 mg |

Nicorandil (60.0 g), after sieving on a 30 mesh sieve was accurately suspended in dimethicone V 100 mm²/s (100 cSt) (240.0 g) and the mixture was stirred to homogeneity. The weight of the obtained mixture was about 300.0 grams. The suspension then obtained was loaded, under constant stirring, into the feeding tank of the dosing loop for liquid suspensions of a machine for the production of soft gelatin capsules. The soft gelatin capsules were filled with the suspension following well known procedures in capsules number 2, shape oval. The soft gelatin capsules obtained (No. 2,765 / yield 92.16 %) were collected in stainless steel trays and stabilized (dehydrated) using conventional methods. The soft gelatin capsules were then preserved in amber glass bottles, with screw cap, at the temperature of 40°C for 180 days and analysed at 90 days and at the end of the storage period.

Reference tablets (about 1,000 tablets) were prepared using conventional methods and the following conventional formulation

| | |
|---|---|
| - nicorandil | 20.000 mg |
| - lactose | 65.000 mg |
| - starch | 9.000 mg |
| - Croscarmellose sodium | 5.000 mg |
| - magnesium stearate | 1.000 mg |
| Total weight of the tablet | 100.000 mg |

Lactose (65.0 g), and croscarmellose sodium (5.0 g) were accurately mixed in mortar and then kneaded with starch glue (9.0 g in about 15.0 ml of water). The wet mixture was granulated by the means of a 30 mesh screen and then dried at about 50°C for about 3 hours. The dried granulate was sieved on a 30 mesh sieve, in order to obtain a uniform granulometry.

Nicorandil (20.0 g) and magnesium stearate (1.0 g) were added and mixed in a polyethylene bag and the mixture was compressed directly with a suitable punch (pressure about 1000 kg) in order to obtain tablets having a mean unitary weight of about 100.0 mg. The reference tablets (No. 952 / yield 95.2%) were packed in separate amber glass bottles, with screw cap, maintained at 40°C for 180 days and analysed at 90 days and at the end of the storage period.

The assay of nicorandil in the samples of soft gelatin capsules according to the invention and in the reference tablets was determined by a suitable high pressure liquid chromatographic method, in order to determine the content of nicorandil during and at the end of the storage period so as to compare the stability of the two formulations. The quantity of nicorandil expressed as a % of the initial assay (considered as 100%), was determined after the production but, before the storage period. The results are set out in the following Table 3. The results establish that the soft gelatin capsules of the invention showed an appreciable stability during the considered storage period and the stability was much higher than the normal reference tablets, preserved at the same conditions.

**TABLE 3**

| Stability study of soft gelatin capsules and of the reference tablets of Example 3, dosed at 20 mg of nicorandil, expressed as assay % of the initial figure (mean figure obtained from the determination of 10 units of each pharmaceutical form). | | | |
|---|---|---|---|
| Compositions | Initial (% theoretic) | Intervals | |
| | | 90 days | 180 days |
| Soft gelatin capsules | 99.63 | 99.21 | 98.94 |
| Reference tablets | 99.77 | 83.14 | 65.88 |

## Claims

1. A pharmaceutical composition suitable for oral or sub-lingual administration comprising a suspension or dispersion of nicorandil in one or more polydimethyl siloxanes.

2. A pharmaceutical composition as claimed in claim 1, where polydimethyl siloxane is a linear polymer with a degree of polymerization of from 20 to 400 and a viscosity at 25ºC of from 20 to 1000 mm²/S.

3. A pharmaceutical composition as claimed in claim 2 wherein the degree of polymerization is from 30 to 100.

4. A pharmaceutical composition as claimed in claim 3 wherein nicorandil comprises from 5% to 20% by weight of the composition.

5. A pharmaceutical composition as claimed in any preceding claim further comprising from 0.5% to 20% colloidal silica by weight of the composition.

6. A pharmaceutical composition as claimed in claim 5 wherein the colloidal silica comprises from 1% to 10% by weight of the composition.

7. A pharmaceutical composition as claimed in any preceding claim further comprising from 1% to 10% lecithin by weight of the composition.

8. A pharmaceutical composition as claimed in any preceding claim wherein the suspension or dispersion is contained within hard or soft gelatin capsules which may optionally include a sealing band.

9. A pharmaceutical composition as claimed in any preceding claim further comprising one or more sweetening agent, flavouring agent and/or essence, each of which is chemically compatible with nicorandil.

10. A pharmaceutical composition as claimed in claim 9 wherein the sweetening agent comprises one or more of saccharose, cyclamate and its salts, saccharin and its salts or aspartame.

11. A pharmaceutical composition as claimed in any preceding claim suitable for sub-lingual administration.

12. Gelatin capsules containing a pharmaceutical 11 composition as described in any one of claims 1 to 11 in which 5 mg, 10 mg or 20 mg of nicorandil is present in each capsule.

13. Use of a composition comprising a suspension or dispersion of nicorandil in one or more polydimethyl siloxanes for the manufacture of a medicament for oral or sub-lingual administration for enhancing coronary vasodilation and suppressing coronary vasoconstriction, especially for treating angina pectoris.

## Patentansprüche

1. Pharmazeutische Zusammensetzung, die für orale oder sublinguale Verabreichung geeignet ist, enthaltend eine Suspension oder Dispersion von Nicorandil in einem oder mehreren Polydimethyl-Siloxanen.

2. Pharmazeutische Zusammensetzung nach Anspruch 1,
bei der das Polydimethyl-Siloxan ein lineares Polymer mit einem Polymerisationsgrad von 20 bis 400 und einer Viskosität bei 25°C von 20 bis 1000 mm²/S ist.

3. Pharmazeutische Zusammensetzung nach Anspruch 2,
bei der der Polymerisationsgrad 30 bis 100 beträgt.

4. Pharmazeutische Zusammensetzung nach Anspruch 3,
bei der das Nicorandil 5 Gew% bis 20 Gew% der Zusammensetzung ausmacht.

5. Pharmazeutische Zusammensetzung nach einem der vorangehenden Ansprüche,
die ferner 0,5% bis 20% kolloidales Siliziumoxid, bezogen auf das Gewicht der Zusammensetzung, enthält.

6. Pharmazeutische Zusammensetzung nach Anspruch 5,
bei der das kolloidale Siliziumoxid 1% bis 10% des Gewichtes der Zusammensetzung ausmacht.

7. Pharmazeutische Zusammensetzung nach einem vorangehenden Anspruch,
die ferner 1% bis 10% Lecithin, bezogen auf das Gewicht der Zusammensetzung, enthält.

8. Pharmazeutische Zusammensetzung nach einem vorangehenden Anspruch,
bei der die Suspension oder Dispersion in harten oder weichen Gelatinekapseln enthalten ist, die ggf. ein Verschlußband enthalten können.

9. Pharmazeutische Zusammensetzung nach einem vorangehenden Anspruch,
die ferner eines oder mehrere Süßmittel, Geschmacksmittel und/oder Geruchsmittel enthält, die jeweils mit Nicorandil chemisch kompatibel sind.

10. Pharmazeutische Zusammensetzung nach Anspruch 9,
bei der das Süßmittel eines oder mehrere von Saccharose, Zyclomat und seinen Salzen, Saccharin und seinen Salzen, oder Aspartam umfaßt.

11. Pharmazeutische Zusammensetzung nach einem vorangehenden Anspruch,
die für Sub-Linguale Verabreichung geeignet ist.

12. Gelatinekapseln, enthaltend eine pharmazeutische Zusammensetzung nach einem der Ansprüche 1 bis 11,
wobei in jeder Kapsel 5 mg, 10 mg oder 20 mg Nicorandil enthalten sind.

13. Verwendung einer Zusammensetzung, die eine Suspension oder Dispersion von Nicorandil in einem oder mehreren Polydimethyl-Siloxanen enthält, für die Herstellung eines Medikaments für orale oder sub-linguale Verabreichung für die Förderung von koronarer Gefäßerweiterung und Unterdrückung von koronarer Gefäßverengung, insbesondere für die Behandlung von Angina Pectoris.

## Revendications

1. Composition pharmaceutique apte à l'administration orale ou sublinguale, comprenant une suspension ou dispersion de nicorandil dans un ou plusieurs polydiméthylsiloxanes.

2. Composition pharmaceutique suivant la revendication 1, dans laquelle le polydiméthylsiloxane est un polymère linéaire ayant un degré de polymérisation de 20 à 400 et une viscosité à 25°C comprise dans l'intervalle de 20 à 1000 mm²/s.

3. Composition pharmaceutique suivant la revendication 2, dans laquelle le degré de polymérisation est compris dans l'intervalle de 30 à 100.

4. Composition pharmaceutique suivant la revendication 3, dans laquelle le nicorandil représente 5 % à 30 % en poids de la composition.

5. Composition pharmaceutique suivant l'une quelconque des revendications précédentes, comprenant en outre 0,5 % à 20 % de silice colloïdale en poids de la composition.

6. Composition pharmaceutique suivant la revendication 5, dans laquelle la silice colloïdale représente 1 % à 10 % en poids de la composition.

7. Composition pharmaceutique suivant l'une quelconque des revendications précédentes, comprenant en outre 1 % à 10 % de lécithine en poids de la composition.

8. Composition pharmaceutique suivant l'une quelconque des revendications précédentes, dans laquelle la suspension ou dispersion est conditionnée dans des gélules ou des capsules de gélatine molle pouvant comprendre facultativement une bande d'étanchéité.

9. Composition pharmaceutique suivant l'une quelconque des revendications précédentes, comprenant en outre un ou plusieurs agents édulcorants ou agents aromatisants et/ou une ou plusieurs essences, chacune de ces substances étant chimiquement compatible avec le nicorandil.

10. Composition pharmaceutique suivant la revendication 9, dans laquelle l'agent édulcorant comprend un ou plusieurs des agents consistant en saccharose, cyclamate et ses sels, saccharine et ses sels ou aspartame.

11. Composition pharmaceutique suivant l'une quelconque des revendications précédentes, apte à l'administration sublinguale.

12. Capsules de gélatine contenant une composition pharmaceutique répondant a la description figurant dans l'une quelconque des revendications 1 à 11, dans lesquelles une quantité de 5 mg, 10 mg ou 20 mg de nicorandil est présente dans chaque capsule.

13. Utilisation d'une composition comprenant une suspension ou dispersion de nicorandil dans un ou plusieurs polydiméthylsiloxanes pour la production d'un médicament destiné à l'administration orale ou sublinguale pour augmenter la vasodilatation des artères coronaires et supprimer la vasoconstriction des artères coronaires, notamment pour le traitement de l'angine de poitrine.
